Europäisches Patentamt

(19) European Patent Office

Office européen des brevets

(11) Publication number: **0 194 401**
**B1**

# (12) EUROPEAN PATENT SPECIFICATION

(45) Date of publication of the patent specification:
**19.07.89**

(21) Application number: **85850414.5**

(22) Date of filing: **23.12.85**

(51) Int. Cl.⁴: **C12N 11/00, B01J 20/28**

(54) Biological method and matrix of mineral wool therefor.

(30) Priority: **17.01.85 SE 8500207**

(43) Date of publication of application:
**17.09.86 Bulletin 86/38**

(45) Publication of the grant of the patent:
**19.07.89 Bulletin 89/29**

(84) Designated Contracting States:
**AT BE CH DE FR GB IT LI LU NL SE**

(56) References cited:
**WO-A-85/01743**
**SE-B- 413 178**
**SE-B- 441 837**
**SE-B- 7 502 057**

(73) Proprietor: **GEDEVELOP AKTIEBOLAG, Fack,
S-260 50 Billesholm(SE)**

(72) Inventor: **Cederberg, Nils Erik Anders, Slöjdgatan 16,
S-260 50 Billesholm(SE)**
Inventor: **Tollemar, Ulf, Tollemar, Ulf,
S-252 23 Helsingborg(SE)**

(74) Representative: **Wiklund, Erik et al, AWAPATENT AB
Box 5117, S-200 71 Malmö(SE)**

ACTORUM AG

## Description

The present invention relates to a biological method and a matrix for carrying out the method.

As used herein, the expression "biological method" refers to such methods as include a "biological catalyst", such as bacteria, yeast, fungi, algae, animal cells, human cells, plant cells, proteins and enzymes. In the following, such biological catalysts are designated "catalyst".

There are two types of biological methods, viz. such as are carried out with the addition of oxygen (aerobic methods) and such as are carried out without the addition of oxygen (anaerobic methods). In methods of the former type, a substrate solution and an oxygen-containing gas, such as air, oxygen-enriched air or pure oxygen, are supplied to a catalyst which then grows and multiplies itself and/or produces metabolites. In methods of the latter type (anaerobic), no oxygen-containing gas is supplied.

The desired product may be either the catalyst itself or a portion thereof, as in single-cell protein production, an extracellular metabolite from the catalyst, such as penicillin in the production of penicillin, or an intracellular metabolite in the catalyst, such as growth hormone in the production of growth hormones, or the process may imply removal of a substance in the substrate solution, such as sewage treatment by biological degradation, or a combination thereof.

Different biological processes are so notorious that an enumeration or detailed description thereof is superfluous here. The conditions for the biological method, such as the composition of the substrate solution, pressure, temperature etc, are established in a manner which is known per se for conventional biological methods. Thus, for instance, the method is normally carried out at ambient pressure, the temperature being about 0-100°C, usually 20-80°, and preferably 20-40°C.

The degree of productivity in aerobic biological processes is generally determined by the size of the available interface of the various phases: oxygen-containing gas (gaseous phase)/substrate solution (liquid phase)/catalyst (solid phase). The larger the available interface, the higher the productivity. Also in anaerobic processes, the productivity is dependent on the available interface, however with the difference that no oxygen-containing gas is present. Almost without exception, the cell concentration or the productivity is desired to be as high as possible in biological methods.

In order that the biological process should proceed as favourably as possible, it is of importance that the supply of substrate solution and (in aerobic processes) oxygen-containing gas (for the sake of simplicity, referred to as air hereinbelow) is efficient. In aerobic processes, one generally tries to achieve this by introducing the catalyst in the substrate solution and oxygenating the substrate solution by letting air in a finely divided state bubble through the substrate solution. The degree of fine division of the air and, thus, the oxygen transfer efficiency are determined substantially by the intensity of agitation in the bio-reactor. An upper limit for the intensity of agitation is set by the physiology of the catalyst. All organisms, and in particular fungi and bacteria producing mycelium, animal cells and plant cells are sensitive to the shear stresses which appear upon intense agitation. Another very common technique is to place the catalyst on an inert carrier, such as crushed slag, macadam, pumice etc, and have the substrate solution flush a bed of the catalyst coated on the carrier while aerating the bed. One example of this are the biological beds which are used for treating sewage. In these prior art methods, it is difficult to achieve an optimally efficient addition of oxygen to the catalyst. In fact, in order that the catalyst should be able to use the oxygen, this must diffuse through the liquid phase of substrate solution surrounding the catalyst. Such diffusion through the liquid phase normally is very slow and is the stage which determines the velocity of the entire process. Therefore, several attempts have been made to facilitate and accelerate the oxygen transfer, for instance by agitation, finely dividing the air, finely dividing the substrate solution into droplets, etc. Although these measures give a certain improvement, they require relatively large amounts of energy.

For instance, the cost of aeration often is, next to costs for raw material and equipment, the largest cost in industrial biological methods. Moreover, the fine division effected to increase the liquid-gas contact surface generally is not very efficient. Thus, even if the substrate solution is finely divided into droplets, each droplet has a substantial mass or bulk into which the air can diffuse entirely only after a relatively long period of time.

It appears from the above that the oxygen-transferring capacity of the system which is decisive of the velocity of aerobic biological methods is not optimal in systems hitherto used, which is a serious drawback, among other things because it sets an upper limit to final cell concentration or productivity in the substrate. In biological methods, it is desirable almost without exception to have this concentration or productivity as high as possible.

It is therefore a primary object of the invention to achieve a biological method in which these drawbacks are obviated and which ensures an efficient supply of substrate solution and, in aerobic methods, air in a manner which is gentle on the catalyst.

It is a further object of the invention to provide a matrix with which the method can be successfully carried out.

These objects are achieved in that it has been discovered in the present invention that the productivity in biological methods is surprisingly improved if a mineral wool matrix with highly orientated fibres is used as the matrix for the catalyst, the substrate solution being permitted to pass through the matrix in the main orientation plane of the fibres.

The reason for the unexpected increase of productivity upon use of a highly orientated mineral wool matrix in the above manner is not fully explained, but to all appearances the reason seems to be that the fibre orientation causes the catalysts to adhere to the matrix in the form of thin films between intersecting fibres, particularly at the points of intersection of the fibres. These films have a thickness of but a very limited number of catalysts, which implies that the contact surface or interface to the substrate solution and air, respectively, is very large and, as mentioned above, the productivity increases as the available interface increases. The reason for the catalysts adhering to the matrix in the form of thin films between the fibres in the present invention has not been established, and normally it would be expected that the catalysts should adhere in larger or smaller lump-shaped aggregates. In such lump-shaped aggregates, the catalysts inside the aggregates are poorly accessible to the substrate solution and air, respectively, i.e. the available interface is considerably smaller than the one according to the invention.

One condition in order to achieve the effect of the invention is that the matrix is a mineral wool matrix having the above-mentioned high-grade fibre orientation, and that, when the method according to the invention is carried out, the substrate solution is permitted to pass through the matrix in the main orientation plane of the fibres.

Thus, the invention provides a biological method in which a biological catalyst immobilised in a fluid-permeable matrix is supplied with a substrate solution and, in an aerobic method, an oxygen-containing gas, the method being characterised in that the biological catalyst is immobilised in a matrix of mineral wool, the fibres of which have a main orientation plane, at least 60% of the total fibre length of the matrix deviating by a maximum of 20° from the main orientation plane, and that the substrate solution is supplied so as to pass through the matrix in the main orientation plane.

Furthermore, the invention provides a fluid-permeable matrix for carrying out the method, which matrix is characterised in that it consists of mineral wool, the fibres of which have a main orientation plane, at least 60% of the total fibre length of the matrix deviating by a maximum of 20° from the main orientation plane.

Further characteristic features of the invention are defined by the dependent claims.

It should here be mentioned that using a matrix of e.g. mineral wool as a biological bed for water treatment is per se previously known and disclosed by, for example, SE patent applications 7308380-0 and 7502057-8.

The present invention uses a fibre matrix of mineral wool, preferably glass wool. Such fibre matrices have all the properties which are required in a matrix according to the present invention, such as inertness, low resistance to gas and liquid flow, good dimensional stability etc, and can also be manufactured at a low cost.

In a matrix of glass wool with a density of 23 kg/m$^3$ which is a normal value for a glass-wool panel for building purposes, the volume of the matrix consists of about 1% glass and 99% voids.

The fibres of the matrix have an average diameter of about 1-500 μm, preferably about 1-100 μm, and most preferably about 1-20 μm.

According to the invention, the fibres of the mineral wool matrix have a main orientation plane, at least 60% of the total fibre length of the matrix deviating by a maximum of 20° from the main orientation plane. Preferably, the deviation does not exceed 20° in at least 70%, and preferably at least 80% of the total fibre length of the matrix.

The main orientation plane of the fibres in the mineral wool matrix according to the invention is provided for instance in that on manufacture of the matrix the velocity is increased as compared to the normal velocity of the substrate on which the fibres are laid, the plane of the substrate thereby being equivalent to the main orientation plane of the matrix fibres. However, the invention is not limited to any particular method of achieving the dominating fibre orientation, but each matrix which has the relevant main orientation plane and in which at least 60% of the total fibre length deviate by a maximum of 20° from the main orientation plane is comprised regardless of the manufacturing method.

As mentioned above, the matrix according to the present invention is three-dimensional, which means that it has an extent in each of three planes at right angles to each other of at least 10 times the fibre diameter. In order to increase the self-supporting capacity of the three-dimensional fibre matrix, the fibres of the matrix at their points of intersection may be linked together by chemical or mechanical bonds. One example of chemical bonding is interconnecting the fibres at their points of intersection by means of polymer binders, for instance of the phenolic resin type. Another example of bonding is fusing the fibres at their points of intersection by heat or by means of a solvent. An example of mechanical bonding is needling the fibre material. A three-dimensional matrix thus bonded is substantially self-supporting, which means that a particular equipment for encapsulating the matrix is normally not required. It may, however, be desirable or suitable in some cases to provide the matrix element with external support means which may, however, be designed in a simple and inexpensive way as gas-permeable walls of e.g. wire netting or perforated metal sheets.

In its simplest embodiment, the matrix consists of a homogeneous fibre body, i.e. of fibres having substantially the same size and properties. In order to counteract penetration of liquid from the downwardly flowing liquid at the vertical boundary walls of the matrix, the outer vertical surfaces of the matrix may be made hydrophobic by treating the fibres in these outer surfaces with hydrophobating oils, waxes or polymers in a per se known way. In these outer layers, the fibres are thus not wetted by the liquid, for which reason the resistance to liquid penetration is high while the gas pressure drop is maintained low. This means that the outer layers serve as an outer boundary to the inner wetted layers of the matrix and allow the gas to pass therethrough, whereas not the liquid.

Further alternative embodiments of the matrix according to the invention include multi-layer matrices in which the matrix body is composed of a plurality of different fibre layers which are distinct or continuously merging into each other and which differ by their fibre diameter, distribution of fibre diameter, fibre length, density etc. These fibre layers are suitably arranged in parallel beside each other or concentrically around each other in the direction of flow of liquid. In the case of distinct fibre layers, the layers may either engage each other directly or be separated by intermediate layers which are preferably hydrophobic.

When carrying out the method according to the invention, the catalyst is first introduced in the fibre matrix, i.e. the catalyst is "grafted" into the matrix.

This is effected in such a way that the catalyst as such or suspended in a suitable liquid is supplied to the fibre matrix and retained therein by the filter action thereof.

Both the initial retention and the subsequent retention of any newly produced cells may, in addition to a pure "screening action" (mechanical retention), also consist of other kinds of interaction between the catalyst and the fibre, such as electric forces of attraction, chemical bonds, phenomena of adhesion pertaining to surface chemistry or physics, flow phenomena and ongrowth.

It is not certain that all catalysts will immediately adhere to and be retained in the matrix. If so, the liquid is suitably recirculated with the suspended catalyst and reintroduced in the matrix. This recirculation is repeated until the desired degree of grafting the catalyst into the matrix has been obtained.

The liquid in which the catalyst is suspended when grafting is performed may consist either of a complete substrate solution which contains all the nutrients essential to the growth of the catalyst, or of an incomplete substrate solution which lacks one or more nutrients, whereby the desired metabolic activity of the catalyst is maintained while its growth is counteracted.

When the catalysts have been grafted into the matrix, the biological method is carried out by supplying the substrate solution and, in the case of an aerobic process, air through the matrix, the substrate solution being supplied in the direction of the main orientation plane. This is preferably carried out in such a manner that the main orientation plane is vertically arranged in the matrix, whereafter the substrate solution is supplied to the matrix from above and penetrates it by gravity. It is, however, appreciated that supplying the substrate and positioning the matrix can also be carried out in other ways as long as the condition that the substrate solution passes through the matrix in the main orientation plane is complied with. The air flow occurring in aerobic processes can be arranged in optional manner in relation to the flow of substrate solution, such as cross-flow, counter-flow or co-flow, cross-flow however being preferred at present. Optionally, also the air supply can be arranged in that the substrate solution is saturated by air before being supplied to the matrix, i.e. there is no specific air flow to the matrix.

In order to facilitate the understanding of the invention and its advantages, an illustrative but non-restrictive Example is given below.

EXAMPLE

The bacterium Kleibsiella pneumoniae ATCC 15380 stored in agar slants with nutrient agar (Difco) was grafted to 200 ml growth medium in a 500 ml E-flask with a plug of cotton wool. The growth medium was composed as follows:

| | |
|---|---|
| Glucose | 10 g/litre |
| (NH$_4$)$_2$SO$_4$ | 5.5 g/litre |
| KH$_2$PO$_4$ | 2.0 g/litre |
| Na$_2$HPO$_4$ | 2.0 g/litre |
| MgSO$_4$ . 7H$_2$O | 0.4 g/litre |
| Tracer element solution containing CaCl$_2$, FeSO$_4$, ZnSO$_4$, Na$_2$MoO$_4$, CoCl$_2$, MnSO$_4$, Na$_2$B$_4$O$_7$ pH adjusted to 7.0 | 1 ml/litre |

The E-flask was placed on a shaking table at 37°C and agitated at 200 r/min, in a 30 mm circulating movement. At the end of the logarithmic growth phase, K. pneumoniae was collected, and 40 ml of a cell suspension with a turbidity of 6.0 at 620 nm were grafted in a reactor which contained 360 ml of the growth medium described above.

The reactor comprised a glass wool matrix with a volume of 0.36 l and with the main orientation plane of the glass wool fibres vertically arranged. The reactor was provided with equipment for supply of liquid at the top of the reactor and for removal or recirculation of liquid at the bottom of the reactor. The reactor was further provided with equipment for conducting air through the reactor in a substantially horizontal flow transverse of the direction of the liquid flow in the reactor. It was possible to measure the oxygen content in the ingoing and outgoing air in order to determine the amount of oxygen which had been consumed in the reactor.

The reactor was first operated for 6 h as a batch reactor while K. pneumoniae was allowed to grow and consume the growth medium. In this batch stage, the conditions in the reactor were as follows:

| | |
|---|---|
| Air flow through the reactor | 0.5 l/min |
| Recirculation flow | 200 ml/min |
| Recirculated volume of medium | 400 ml |
| Temperature | 37°C |
| pH | 7.0 |

While maintaining the above-mentioned conditions of the recirculation, a continuous flow through the reactor of a lean medium was started after 6 h, said lean medium consisting of the above-mentioned growth medium in which, however, the concentration of the components included had been reduced to 1/10. The reactor was here used as a chemostat with a dilution rate of 1.25 h$^{-1}$. After 48 h, the oxygen absorption rate per unit of volume of the reactor was measured.

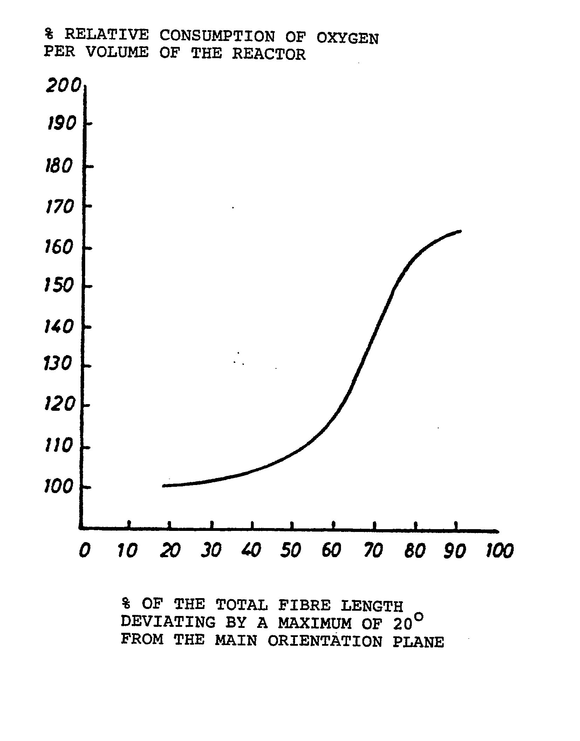

The above experiment of measuring the oxygen absorption rate per unit of volume of the reactor was repeated, but with the difference that the glass wool matrix was replaced by other glass wool matrices having another degree of fibre orientation. A measure of the fibre orientation was indicated by the percentage of the total fibre length in the matrix, which deviates by a maximum of 20° from the main orientation plane of the fibres in the matrix. By plotting the relative consumption of oxygen (in percentages) per unit of volume of the reactor as a function of the degree of fibre orientation of the matrix expressed in percentage of the total fibre length which deviates by a maximum of 20° from the main orientation plane of the fibres, the graph in Fig. 1 was obtained. The graph clearly indicates that a pronounced increase of the relative consumption of oxygen, i.e. an improvement of the efficiency of the reactor, occurs when the fibres of the matrix are strongly oriented in the main orientation plane, more precisely when at least 60% of the total fibre length deviate by a maximum of 20° from the main orientation plane of the fibres in the matrix (the direction of liquid flow through the matrix).

## Claims

1. A biological method in which a biological catalyst immobilised in a fluid-permeable matrix is supplied with a substrate solution and, in an aerobic method, an oxygen-containing gas, **characterised** in that the biological catalyst is immobilised in a matrix of mineral wool, the fibres of which have a main orientation plane, at least 60% of the total fibre length of the matrix deviating by a maximum of 20° from the main orientation plane, and that the substrate solution is supplied so as to pass through the matrix in the main orientation plane.

2. A method as claimed in claim 1, **characterised** in that at least 70% of the total fibre length of the matrix deviate by a maximum of 20° from the main orientation plane.

3. A method as claimed in claim 2, **characterised** in that at least 80% of the total fibre length of the matrix deviate by a maximum of 20° from the main orientation plane.

4. A fluid-permeable matrix for carrying out the method according to any one of claims 1-3, **characterised** in that it consists of mineral wool, the fibres of which have a main orientation plane, at least 60% of the total fibre length of the matrix deviating by a maximum of 20° from the main orientation plane.

5. A matrix as claimed in claim 4, **characterised** in that at least 70% of the total fibre length of the matrix deviate by a maximum of 20° from the main orientation plane.

6. A matrix as claimed in claim 5, **characterised** in that at least 80% of the total fibre length of the matrix deviate by a maximum of 20° from the main orientation plane.

## Patentansprüche

1. Biologische Methode, bei der einem in einer fluiddurchlässigen Matrize immobilisierten, biologischen Katalysator eine Substratlösung zugesetzt wird und, in einer aeroben Methode, ein Sauerstoff enthaltendes Gas, dadurch gekennzeichnet, daß der biologische Katalysator in einer Matrize aus Schlackenwolle immobilisiert wird, deren Fasern eine Hauptorientierungsebene aufweisen, wobei zumindest 60% der gesamten Faserlänge der Matrize um höchstens 20° von der Hauptorientierungsebene abweichen, und daß die Substratlösung in einer solchen Weise zugesetzt wird, daß sie die Matrize in der Hauptorientierungsebene passiert.

2. Methode nach Anspruch 1, dadurch gekennzeichnet, daß zumindest 70% der gesamten Faserlänge der Matrize um höchstens 20° von der Hauptorientierungsebene abweichen.

3. Methode nach Anspruch 2, dadurch gekennzeichnet, daß zumindest 80% der gesamten Faserlänge der Matrize um höchstens 20° von der Hauptorientierungsebene abweichen.

4. Fluiddurchlässige Matrize zur Durchführung der Methode nach einem der Ansprüche 1–3, dadurch gekennzeichnet, daß sie aus Schlackenwolle besteht, deren Fasern eine Hauptorientierungsebene aufweisen, wobei zumindest 60% der gesamten Faserlänge der Matrize um höchstens 20° von der Hauptorientierungsebene abweichen.

5. Matrize nach Anspruch 4, dadurch gekennzeichnet, daß zumindest 70% der gesamten Faserlänge der Matrize um höchstens 20° von der Hauptorientierungsebene abweichen.

6. Matrize nach Anspruch 5, dadurch gekennzeichnet, daß zumindest 80% der gesamten Faserlänge der Matrize um höchstens 20° von der Hauptorientierungsebene abweichen.

## Revendications

1. Méthode biologique dans laquelle on ajoute à un catalyseur biologique immobilisé dans une matrice perméable aux fluides une solution de substrat et, dans une méthode aérobie, un gaz contenant l'oxygène, caractérisée par le fait que le catalyseur biologique est immobilisé dans une matrice de laine minérale dont les fibres présentent un plan d'orientation principal, 60% au moins de la longueur totale des fibres de la matrice s'écartant de 20° au maximum dudit plan d'orientation principal, et que la solution de substrat est ajoutée de manière à passer par la matrice dans le plan d'orientation principal.

2. Méthode selon la revendication 1, caractérisée par le fait que 70% au moins de la longueur totale des fibres de la matrice s'écartent de 20° au maximum dudit plan d'orientation principal.

3. Méthode selon la revendication 2, caractérisée par le fait que 80% au moins de la longueur totale des fibres de la matrice s'écartent de 20° au maximum dudit plan d'orientation principal.

4. Matrice perméable aux fluides pour la mise en œuvre de la méthode selon l'une quelconque des revendications 1 à 3, caractérisée par le fait qu'elle consiste en laine minérale dont les fibres présentent un plan d'orientation principal, 60% au moins de la longueur totale des fibres de la matrice s'écartant de 20° au maximum dudit plan d'orientation principal.

5. Matrice selon la revendication 4, caractérisée par le fait que 70% au moins de la longueur totale des fribres de la matrice s'écartent de 20° au maximum dudit plan d'orientation principal.

6. Matrice selon la revendication 5, caractérisée par le fait que 80% au moins de la longueur totale des fibres de la matrice s'écartent de 20° au maximum dudit plan d'orientation principal.

% RELATIVE CONSUMPTION OF OXYGEN
PER VOLUME OF THE REACTOR

% OF THE TOTAL FIBRE LENGTH
DEVIATING BY A MAXIMUM OF 20°
FROM THE MAIN ORIENTATION PLANE